# EUROPEAN PATENT APPLICATION

(11) **EP 1 949 889 A1**
(43) Date of publication of application: **30.07.2008**
(21) Application number: 06811869.4
(22) Date of filing: 17.10.2006
(51) Int. Cl.: A61K 8/97, A61K 8/49, A61K 36/18, A61P 17/02, A61P 17/16, A61P 43/00, A61L 27/00

(54) **FIBROBLAST ACTIVATOR, METHOD FOR ACTIVATION OF FIBROBLAST, COLLAGEN SYNTHESIS PROMOTER, METHOD FOR PROMOTION OF COLLAGEN SYNTHESIS, SKIN AGING-PREVENTING AGENT, AND METHOD FOR PREVENTION OF AGING OF THE SKIN**

(30) Priority: 18.10.2005 JP 2005302591
(71) Applicant: MERCIAN CORPORATION, Chuo-ku, Tokyo 104-8305 (JP)
(72) Inventor: WATANABE, Azuma, Kanagawa 2510057 (JP); IMAMURA, Kazuyuki, Kanagawa 2100818 (JP)
(74) Representative: Patentanwälte Ruff, Wilhelm, Beier, Dauster & Partner
(86) International application number: PCT/JP2006/320614
(87) International publication number: WO 2007/046353

(57) **Abstract**

The present invention relates to a fibroblast activator comprising grape sap as an active ingredient, a method of activating a fibroblast, using grape sap, and a method of using grape sap as a fibroblast activator. The present invention further relates to a collagen synthesis promoter comprising grape sap as an active ingredient, a method of promoting collagen synthesis using grape sap, and a method of using grape sap as a collagen synthesis promoter. The present invention further relates to a skin antiaging agent comprising grape sap as an active ingredient, a method of preventing aging of skin using grape sap, and a method of using grape sap as a skin antiaging agent. The present invention provides highly safe and effective means of preventing aging.

## Description

### TECHNICAL FIELD

The present invention relates to a fibroblast activator that activates fibroblasts participating in the decomposition and synthesis of collagen, a method of activating a fibroblast, a collagen synthesis promoter, a method of promoting collagen synthesis, a skin antiaging agent capable of preventing aging of skin by activating fibroblasts and promoting collagen synthesis, and a method of skin antiaging.

### BACKGROUND TECHNIQUE

Generally, aging of skin is the physiological phenomenon that is produced by the combined effects of physiological aging with the advancement of age and photoaging caused by ultraviolet radiation. Upon entering one's 20's or 30's, proteins in the skin, such as the collagen that is the source of skin elasticity in the dermal layer, decrease with age. The strength of the collagen-producing fibroblasts themselves also decreases. Photoaging is a phenomenon whereby enzymes that decompose collagen are secreted due to exposure to ultraviolet radiation of the skin, thereby damaging the collagen and causing loss of dermal elasticity. Such reduction in the quantity of collagen and diminished functioning of fibroblasts are major causes of aging phenomena such as wrinkling and sagging.

Accordingly, countermeasures to aging have been proposed in the form of antiaging components that inhibit the activation of collagen decomposing enzyme (collagenase). For example, collagenase-blocking agents in the form of a certain mushroom extract (Japanese Unexamined Patent Publication (KOKAI) Heisei No. 9-40552); extracts of plants of the genus *Artemisia* (Japanese Unexamined Patent Publication (KOKAI) Heisei No. 10-194982); extracts of grape seeds, apple seeds, and herbaceous plants of the family *Saxifragaceae* (Japanese Unexamined Patent Publication (KOKAI) Heisei No. 11-71294); extracts of plants of the family *Thyme* (Japanese Unexamined Patent Publication (KOKAI) Heisei No. 11-79970); extracts of the eucalyptus tree, salvia, *Houttuynia cordata,* tree peony, peony, and the like (Japanese Unexamined Patent Publication (KOKAI) Heisei No. 11-147833; extracts of hypericum and hypericum perforatum (Japanese Unexamined Patent Publication (KOKAI) Heisei No. 11-315008); extract of *Crocosmia* X *crocosmiiflora* bulbs (Japanese Unexamined Patent Publication (KOKAI) No. 2000-191512); and extracts of catechu, persimmon, burnet, peppermint, and the like (Japanese Unexamined Patent Publication (KOKAI) No. 2000-159631) have been proposed.
However, by simply inhibiting the activation of collagen decomposing enzyme, merely collagen decomposition by ultraviolet radiation (photoaging) is prevented, but skin aging that has undergone advanced due to ultraviolet radiation or increasing age cannot be effectively improved.

Retinolic acid (retinoic acid, a derivative of vitamin A) is well known as an antiaging component that rather effectively promotes the synthesis of collagen itself (Methods in Enzymology, 1990, vol. 190, p. 352-360).
However, retinolic acid is strongly irritating, and it is limited to use by doctors in Japan. Thus, there is need for the development of a highly effective and safe antiaging agent.

### DISCLOSURE OF THE INVENTION

### PROBLEMS TO BE SOLVED BY THE INVENTION

Under these circumstances, it is an object of the present invention to provide a highly safe and effective means of preventing aging.

### MEANS FOR SOLVING PROBLEMS

The present inventors conducted extensive research into achieving the above-stated object, resulting in the discovery that grape sap has a fibroblast-activating function and a function of promoting collagen synthesis by fibroblasts; the present invention was devised on this basis.

The present invention relates to a fibroblast activator comprising grape sap as an active ingredient, a method of activating a fibroblast using grape sap, and a method of using grape sap as a fibroblast activator.

The present invention further relates to a collagen synthesis promoter comprising grape sap as an active ingredient, a method of promoting collagen synthesis using grape sap, and a method of using grape sap as a collagen synthesis promoter.

The present invention further relates to a skin antiaging agent comprising grape sap as an active ingredient, a method of preventing aging of skin using grape sap, and a method of using grape sap as a skin antiaging agent.

### EFFECTS OF THE INVENTION

According to the present invention, aging of the skin can be effectively prevented by promoting the synthesis of collagen. Further, when proline is used in combination with grape sap, the present invention can further strengthen the effect of grape sap.

### BEST MODE FOR CARRYING OUT THE INVENTION

The present invention will be described in more detail below.

The present invention relates to a fibroblast activator comprising grape sap as an active ingredient, and the fibroblast activator of the present invention can comprise proline in addition to grape sap.

The present invention relates to a method of activating a fibroblast using grape sap, and the method of activating a fibroblast of the present invention can use proline in addition to grape sap.

The present invention relates to a method of using grape sap as a fibroblast activator, and in the method of using of the present invention, the fibroblast activator can comprise proline in addition to grape sap.

Collagen is a fibrous protein present in almost all cells, such as skin, blood vessels, tendons and teeth, accounting for about 30 percent of all protein constituting the human body. The human body is comprised of equal to or more than 60 trillion cells. Cells and organs comprised of cells are properly shaped by the extracellular matrix. Collagen is the protein that makes up the extracellular matrix. It serves as a scaffolding for cells, plays roles in the proliferation and functional maintenance of cells, and plays important roles as a constituent component of matrix proteins in cartilage and bone.

Fibroblasts are one of the main types of cells that produce collagen. The collagen that is secreted by fibroblasts fills in the gaps between cells to form the intercellular matrix. As a result of investigation, the present inventors discovered for the first time that grape sap has a function of activating fibroblasts themselves (a fibroblast-activating function).

The use of proline in combination with grape sap can enhance the fibroblast-activating effect possessed by grape sap. The proline is preferably L-proline.

The present invention relates to a collagen synthesis promoter comprising grape sap as an active ingredient, and the collagen synthesis promoter of the present invention can comprise proline in addition to grape sap. The present invention relates to a method of promoting collagen synthesis using grape sap, and the method of promoting collagen synthesis of the present invention can use proline in addition to grape sap. The present invention relates to a method of using grape sap as a collagen synthesis promoter, and in the method of using of the present invention, the collagen synthesis promoter can comprise proline in addition to grape sap.

As set forth above, grape sap has a fibroblast-activating function. The present inventors conducted research resulting in the new discovery that grape sap has the function of promoting collagen synthesis in fibroblasts. The collagen synthesis promoter of the present invention comprises, as an active ingredient, grape sap that works on fibroblasts themselves and has the functions of activating the cells themselves and promoting collagen synthesis by fibroblasts, thereby effectively promoting collagen synthesis.

The use of proline in combination with grape sap can enhance the collagen synthesis promoting effect possessed by grape sap. The proline is preferably L-proline.

The present invention relates to a skin antiaging agent comprising grape sap as an active ingredient, and the skin antiaging agent of the present invention can comprise proline in addition to grape sap. The present invention relates to a method of preventing aging of skin using grape sap, and the method of preventing aging of skin of the present invention can use proline in addition to grape sap. The present invention relates to a method of using grape sap as a skin antiaging agent, and in the method of using of the present invention, the skin antiaging agent can comprise proline in addition to grape sap.

Changes of properties and conditions of collagen, and functions of fibroblasts are significantly involved with aging of skin, such as loss of skin tension and wrinkles. This is because, excluding water, the skin is comprised of roughly 80 percent collagen. The skin is divided into the three layers of, from the surface down, the epidermis, dermis, and subdermal tissue. Among them, collagen is present in the dermis, constituting the greater portion thereof, and has the structure and properties of a textile. Since various forces are applied to the skin from the exterior, the skin is required to be strong and flexible; such properties are mainly due to the functions of collagen. The decomposition and synthesis of collagen are controlled by the fibroblasts, and collagen is maintained in a constant state of tension by the strength of the fibroblasts. The skin antiaging agent of the present invention comprises grape sap as an active ingredient, and its collagen synthesis promoting function and fibroblast activating function can effectively prevent aging of the skin in the form of loss of skin tension and wrinkles.

The use of proline in combination with grape sap can enhance the antiaging effect on skin possessed by grape sap. The proline is preferably L-proline.

Grape sap is tracheary water that flows in the grape plant (family *Vitis).* The type of grape sap employed in the present invention is not specifically limited other than that it be sap of a plant of family *Vitis;* the sap of the grape, *Vitis coignetiae,* or the like may be employed. Examples are grape varieties such as: Kyoho, Koshu, Kaiji, Muscat Berry A, Delaware, Cambell Early, Pione, Neo Muscat, Niagara, Concord, Steuben, Cabernet Sauvignon, Pinot Noir, Merlot, Sémillon, Riesling, and Chardonnay. Neither the region of cultivation, method of cultivation, nor the like is limited. However, sap flowing out of the pruned branches of grape in early spring, before the budding season, is preferable.

Grape sap can be obtained by cutting the stems of grape and collecting the sap that flows out. The method of collecting the sap is not specifically limited. For example, small containers or the like can be attached at the sites of pruning so that the sap flows into them, the sap that flows out can be collected, and these can be collected in a large storage container to which preservatives or the like have been added. The use of a device capable of collecting sap from various locations at once, such as the collection device described in Japanese Unexamined Patent Publication (KOKAI) Heisei No. 10-295207, and the liquid receiving container described in Japanese Unexamined Patent Publication (KOKAI) No. 2005-118035, for collection is preferable because such collection is highly efficient and deterioration in quality is prevented.

As needed, the collected grape sap can be mixed with preservatives or the like and employed in liquid form or as a freeze-dried product. As needed, the collected grape sap can be subjected to a heat or clarification treatment. This prevents the sap from clouding or turning brown. The heat treatment can be conducted by heating the grape sap to, for example, 60 to 100°C, preferably 80 to 90°C, for equal to or longer than 10 minutes, for example. The heat treatment can be conducted on the grape sap after collection, or conducted following processing as a product. The clarification treatment can be conducted using a known clarifying agent, such as activated carbon, bentonite, silica gel, silica sol, or PVPP (polyvinyl polypyrrolidone).

The fibroblast activator, collagen synthesis promoter, and skin antiaging agent of the present invention each can contain grape sap in liquid form or in the form of a freeze-dried product. The formulation is not specifically limited. They can be provided in various formulations such as lotions, emulsions, gels, creams, ointments, powders, and grains. The fibroblast activator, collagen synthesis promoter, and skin antiaging agent of the present invention may each be comprised of just grape sap, or of grape sap and proline. As needed, oil-based components, surfactants, moisturizers, pigments, ultraviolet radiation-absorbing agents, antioxidants, fragrance materials, dyes, antibacterial agents, fungus-combating agents, alcohols, water and the like, that are commonly blended into pharmaceuticals, quasi-drugs, skin cosmetics and the like, may be suitably blended in. To the extent that the effect of the present invention is not lost, other cell activators, collagen synthesis promoters, and antiaging agents may also be used together.

The fibroblast activator, collagen synthesis promoter, and skin antiaging agent of the present invention can comprise grape sap in a proportion of equal to or greater than 1 weight percent, preferably 3 weight percent to 100 weight percent, and more preferably, 10 weight percent to 80 weight percent, based on the unadjusted sap. In the present invention, grape sap itself may be used as a fibroblast activator, collagen synthesis promoter, and skin antiaging agent.

When proline is blended into the fibroblast activator, collagen synthesis promoter, or skin antiaging agent of the present invention in addition to grape sap, the quantity blended in suitably falls within a range of 0.1 to 3 weight percent, preferably 0.1 to 1.5 weight percent. The quantity of proline blended in is given relative to the total of proline and grape sap being100 percent.

The method of applying the fibroblast activator, collagen synthesis promoter, or skin antiaging agent of the present invention as a topical agent to the skin may be suitably selected based on the type of topical agent. Generally, when applying it as a topical agent prepared as a lotion, emulsion, gel, cream, or ointment, approximately one or two applications daily to the facial skin or the like is preferable. The fibroblast activator, collagen synthesis promoter, and skin antiaging agent of the present invention can be employed as cosmetics, and can be applied to lotions, emulsions, beauty solutions, creams, liquid foundations, powder foundations, lipstick, and the like.

The quantity of the fibroblast activator, collagen synthesis promoter, or skin antiaging agent of the present invention administered when employed as an orally administered drug in the form of a powder or grain can be suitably selected based on conditions such as the age and skin condition of the person to whom it is being administered. The quantity administered falls within a range of, for example, 100 to 1,000 mg (as a quantity of active ingredient) for an adult. However, the above-stated administration quantity can be suitably altered based on the above-stated conditions.

### EXAMPLES

The present invention will be described in greater detail below through Examples. However, the present invention is not limited to Examples given below.
The grape sap employed in Examples below was obtained by using the device described in Japanese Unexamined Patent Publication (KOKAI) Heisei No. 10-295207 to collect sap running out of grape (variety: Koshu) branches being trimmed in a vineyard in Katsunuma-cho, Higashi Yamanashi-gun, Yamanashi Prefecture in early April, 2005. One gram of freeze-dried grape sap was equivalent to 280 g of unadjusted sap.

### Example 1

### (Test of fibroblast activation function)

3-(4,5-Dimethyl-2-thiazolyl)-2,5-diphenyl-2H-tetrazolium bromate (MTT) cleaves NADPH or NADH produced in energy metabolic processes in mitochondria within cells to produce formazan. Thus, the quantity of formazan produced was adopted as an indicator of cell activation function.

Normal human fibroblasts were seeded onto a 96-well microplate at a cell density of 3.0 x 10⁴ cells/well. After culturing for 24 hours in Dulbecco's modified eagle medium (DMEM) containing 1 percent fetal bovine serum (FBS), the medium was replaced with DMEM containing 1 percent FBS containing a freeze-dried product of grape sap in the concentration shown in Table 1. A positive control (PC) in the form of DMEM containing 5 percent FBS was employed. After culturing for 48 hours, the medium was replaced with DMEM containing 1 percent FBS containing 0.4 mg/mL MTT (Nacalai) and cultivation was conducted for two hours.

The cells were washed, the formazan produced in the cells was dissolved in 2-propanol, and a microplate reader was employed to measure the absorbance at 550 nm and 650 nm. The difference between the two was calculated to evaluate the quantity of formazan generated within the cells. Cell activation function was denoted by an activation index (%) with the absorbance (ABS.) of cultivated cells to which no sample was added (control) being denoted as 100. The results are shown in Table 1. All the results are given as the average value ± standard deviation. Student's t-test was employed to determine significant differences. The p value was determined to be significant when p < 0.05. As shown in Table 1, cultivation in a medium to which grape sap had been added was determined to activate fibroblasts.

**[Table 1]**

| Cell activation function for fibroblast (n=5) | | | |
|---|---|---|---|
| | Concentration (µg/ml) | Activation index (%) | |
| | | Mean ± SD | P (t-test) |
| Control | - | 100.0 ± 1.7 | 1.000 |
| PC | - | 116.0 ± 2.9 | 0.000 |
| Freeze-dried product of grape sap | 500.0 | 114.6 ± 6.7 | 0.006 |
| | 1000.0 | 120.3 ± 6.9 | 0.001 |

| | | | |
|---|---|---|---|
| Underline: Cell activation function exists. | | | |

### Example 2

### (Test of the collagen synthesis promoting function of fibroblasts)

The collagen synthesis of fibroblasts was evaluated by quantifying the amount of secreted type I collagen in culture supernatant by ELISA.

Normal human fibroblasts were seeded onto a 96-well microplate at a cell density of 3.0 x 10⁴ cells/well. After culturing for 24 hours in DMEM containing 0.5 percent FBS, the medium was replaced with DMEM containing 0.5 percent FBS containing a freeze-dried product of grape sap in the concentration shown in Table 2. A PC in the form of magnesium ascorbyl phosphate (VCPMg) was employed. After 48 hours, the culture supernatant was collected and the amount of type I collagen was quantified by ELISA.

Culture supernatant and calibration curve-use type I collagen were placed in a 96-well plate and coated overnight at 4°C. A 1 percent bovine serum albumin (BSA) solution was used to conduct blocking for 1 hour at 37°C, after which the solution was reacted for 1.5 hours at 37°C with primary antibody (antihuman type I collagen (rabbit)). It was then reacted for 1.5 hours at 37°C with secondary antibody (Histofine PO (rabbit)), after which phosphate-citrate buffer (0.1 M, pH 4.0) containing 0.3 mg/mL of 2,2'-azinobis(3-ethylbenzthiazoline-6-sulfonate)diammonium (ABTS) was added. Twenty minutes later, a microplate reader was employed to measure the absorbance at 405 nm. The quantity of collagen in the culture supernatant was calculated from a calibration curve measured with the same plate.

At the same time, the quantity of cell protein was quantified using BCA protein assay reagent (PIERCE). The quantity of collagen in the culture supernatant was divided by the quantity of protein to calculate the quantity of collagen per unit of protein, which was adopted as the quantity of collagen synthesized by the cells. The results are given in Table 2. All of the results are denoted as the average value ± standard deviation. Student's t-test was employed to determine significant differences. The p value was determined to be significant when p < 0.05. As shown in Table 2, cultivation in a medium containing grape sap was found to promote collagen synthesis by fibroblasts by about 1.2-fold to 3.6-fold that when grape sap was not added (control). The function was particularly marked when medium containing a freeze-dried product of grape sap of equal to or greater than 500 micrograms/mL was employed. When medium containing a freeze-dried product of grape sap of equal to or greater than 250 micrograms/mL was employed, a significant increase in protein quantity was observed. The increase in protein indicated that the fibroblasts had been activated and had proliferated. The quantity of collagen shown in Table 2 is a value that has been divided by the amount of protein, and it can be understood that the collagen synthesis levels of individual cells increased significantly.

**[Table 2]**

| | Concent ration (µg/ml) | Collagen amount (ng/µg protein) | | Protein amount (µg) | |
|---|---|---|---|---|---|
| | | Mean ± SD | p (t-test) | Mean ± SD | p (t-test) |
| Control | - | 12.9 ± 0.4 | 1.000 | 11.0±0.3 | 1.000 |
| PC | - | 32.9 ± 4.8 | 0.000 | 11.2±0.2 | 0.230 |
| Freeze-dried product of grape sap | 125.0 | 15.0 ± 1.1 | 0.003 | 11.1±0.4 | 0.761 |
| | 250.0 | 19.3 ± 1.4 | 0.000 | 11.9±0.3 | 0.001 |
| | 500.0 | 41.9 ± 1.9 | 0.000 | 12.3±0.2 | 0.000 |
| | 1000.0 | 46.0 ± 2.0 | 0.000 | 12.4±0.2 | 0.000 |

| | | | | | |
|---|---|---|---|---|---|
| Underline: Collagen synthesis was significantly promoted. | | | | | |

### Example 3

### (Test of combined use with proline)

### Preparation of test samples

To determine the effects of using proline in combination with grape sap, a cream was formulated as indicated in Table 3 for use in the following organoleptic evaluation tests and wrinkle evaluation tests. All blending quantities of the compositions given in Table 3 are weight percentages.

**[Table 3]**

| Starting materials | Comp.Ex.1 | Ex.1 | Ex.2 | Ex.3 |
|---|---|---|---|---|
| Grape sap | 0.00 | 5.00 | 5.00 | 5.00 |
| L-proline | 1.00 | 0.00 | 0.50 | 1.00 |
| Xanthan gum (2 percent aqueous solution) | 5.00 | 5.00 | 5.00 | 5.00 |
| Polyethylene glycol 1000 | 2.50 | 2.50 | 2.50 | 2.50 |
| Acrylic acid - alkyl methacrylate copolymer (2 percent aqueous solution) | 15.00 | 15.00 | 15.00 | 15.00 |
| Carboxyvinyl polymer (2 percent aqueous solution) | 20.00 | 20.00 | 20.00 | 20.00 |
| Purified water | 33.50 | 29.50 | 29.00 | 28.50 |
| Dipotassium glycyrrhizinate | 0.10 | 0.10 | 0.10 | 0.10 |
| Dipropylene glycol | 3.00 | 3.00 | 3.00 | 3.00 |
| 1,3-Butylene glycol | 5.00 | 5.00 | 5.00 | 5.00 |
| Concentrated glycerin | 4.00 | 4.00 | 4.00 | 4.00 |
| Phenoxyethanol | 0.40 | 0.40 | 0.40 | 0.40 |
| Decaglyceryl monomyristate | 0.50 | 0.50 | 0.50 | 0.50 |
| Triglyceryl 2-ethylhexanoate | 1.00 | 1.00 | 1.00 | 1.00 |
| Vegetable squalane | 2.00 | 2.00 | 2.00 | 2.00 |
| Decamethyl cyclopentasiloxane | 2.00 | 2.00 | 2.00 | 2.00 |
| Methyl polysiloxane | 1.00 | 1.00 | 1.00 | 1.00 |
| Potassium hydride | 0.35 | 0.35 | 0.35 | 0.35 |
| Purified water | 3.65 | 3.65 | 3.65 | 3.65 |
| Total | 100.00 | 100.00 | 100.00 | 100.00 |

### Organoleptic evaluation test

Sixty women aged from 30's to 50's who stated that their skins were dry or lacked suppleness or tension were employed as test subjects. They were divided into four groups of 15 each without deviation in age. The creams of the four formula examples given in Comparative Example 1 and Examples 1 to 3 indicated in Table 3 were given to the respective groups of test subjects, who were requested to use them twice a day, once in the morning and once at night, for 30 consecutive days. An organoleptic evaluation test was conducted on the use sensations: a moist sensation (moisturizing property), tension sensation (resilience), and softness sensation (flexibility). The evaluation criteria are given in Table 4, and the results of the organoleptic evaluation test in Table 5.

**[Table 4]**

| Use sensation: | Evaluation score | Evaluation |
|---|---|---|
| Moist sensation (moisturizing property) | 5 | Very moist |
| | 4 | Moist |
| | 3 | Normal |
| | 2 | Not moist |
| | 1 | Not moist at all |
| | | |
| Tension sensation (resilience) | 5 | Very tensional |
| | 4 | Tensional |
| | 3 | Normal |
| | 2 | Not tensional |
| | 1 | Not tensional at all |
| | | |
| Softness sensation (flexibility) | 5 | Very soft |
| | 4 | Soft |
| | 3 | Normal |
| | 2 | Not soft |
| | 1 | Not soft at all |

**[Table 5]**

| | Comp.Ex.1 | Ex.1 | Ex.2 | Ex.3 |
|---|---|---|---|---|
| Moist sensation (moisturizing property) | 2.9 | 4.1 | 4.2 | 4.6 |
| Tension sensation (resilience) | 2.8 | 3.2 | 3.9 | 4.5 |
| Softness sensation (flexibility) | 2.7 | 3.1 | 3.5 | 4.2 |
| Average value | 2.8 | 3.5 | 3.9 | 4.4 |

| | | | | |
|---|---|---|---|---|
| * Average evaluation score of 15 members for each example, and total average value of three evaluation items. | | | | |

### Wrinkle evaluation test

Forty women aged from 40's to 60's who were concerned about skin wrinkles (particularly small wrinkles at the corner of the eye) were employed as test subjects. They were divided into four groups of 10 each without deviation in age. The creams of the four formula examples given in Comparative Example 1 and Examples 1 to 3 indicated in Table 3 were given to the respective groups of test subjects, who were requested to use them twice a day, once in the morning and once at night, for 30 consecutive days while focusing on the areas around the eyes where wrinkles tend to form. The numbers of wrinkles at the corners of the eyes before and after application were compared by visual determination using UV photography. The results are given in Table 6.

**[Table 6]**

| | Comp.Ex.1 | Ex.1 | Ex.2 | Ex.3 |
|---|---|---|---|---|
| Numbers of wrinkles before application (0 day) | 24 | 22 | 27 | 29 |
| Numbers of wrinkles after application (30 day) | 22 | 17 | 19 | 18 |
| Improvement (Reduction) rate of numbers of wrinkles | 8% | 23% | 30% | 38% |

| | | | | |
|---|---|---|---|---|
| *Average numbers of wrinkles of 10 members for each example, and improvement (reduction rate) before and after application. | | | | |

### Test results

Both in Table 5 showing the organoleptic evaluation test results and in Table 6 showing the wrinkle evaluation test results, the cream containing grape sap of Example 1 afforded better effects in terms of improvement in dryness, tension, wrinkles, and the like than the cream containing proline of Comparative Example 1. Further, the creams of Examples 2 and 3, containing combinations of grape sap and proline, afforded better effects in terms of improving dryness, tension, wrinkles, and the like than either grape sap or proline employed alone. That is, grape sap has an excellent effect in terms of improvement in dryness, tension, wrinkles, and the like. Further, the combining amino acids that constitute collagen, particularly proline, with grape sap for use is found to enhance the moisturizing effect on skin and further enhance the effect of reducing the appearance of wrinkles.

### INDUSTRIAL APPLICABILITY

The present invention can effectively prevent aging of the skin through the fibroblast activation function of grape sap and through the collagen synthesis promoting function of grape sap on fibroblasts.

In addition to preventing aging, the application of the collagen synthesis promoter and the fibroblast activator of the present invention can be anticipated for use on the treatment of pimple marks and pregnancy stretch marks, the treatment of wounds such as burn wounds, promoting artificial skin cultures, and the like.

## Claims

1. A fibroblast activator comprising grape sap as an active ingredient.

2. The fibroblast activator according to claim 1, further comprising proline.

3. A method of activating a fibroblast, using grape sap.

4. The method of activating a fibroblast according to claim 3, using proline together.

5. A method of using grape sap as a fibroblast activator.

6. The method according to claim 5, wherein the fibroblast activator further comprises proline.

7. A collagen synthesis promoter comprising grape sap as an active ingredient.

8. The collagen synthesis promoter according to claim 7, further comprising proline.

9. A method of promoting collagen synthesis, using grape sap.

10. The method of promoting collagen synthesis according to claim 9, using proline together.

11. A method of using grape sap as a collagen synthesis promoter.

12. The method according to claim 11, wherein the collagen synthesis promoter further comprises proline.

13. A skin antiaging agent comprising grape sap as an active ingredient.

14. The skin antiaging agent according to claim 13, further comprising proline.

15. A method of preventing aging of skin, using grape sap.

16. The method of preventing aging of skin, using proline together.

17. A method of using grape sap as a skin antiaging agent.

18. The method according to claim 17, wherein the skin antiaging agent further comprises proline.
